# EUROPEAN PATENT SPECIFICATION

(11) **EP 4 189 388 B1**
(45) Date of publication and mention of the grant of the patent: **08.01.2025**
(21) Application number: 20788914.8
(22) Date of filing: 29.07.2020
(51) Int. Cl.: G01N 33/49, B01L 3/00

(54) **A DEVICE, AN INTEGRATED SYSTEM AND A METHOD FOR DETECTING CIRCULATING TUMOR CELLS**
VORRICHTUNG, INTEGRIERTES SYSTEM UND VERFAHREN ZUR ERKENNUNG ZIRKULIERENDER TUMORZELLEN
DISPOSITIF, SYSTÈME INTÉGRÉ ET PROCÉDÉ POUR LA DÉTECTION DE CELLULES TUMORALES CIRCULANTES

(43) Date of publication of application: 07.06.2023
(73) Proprietor: Sabanci Üniversitesi, 34956 Tuzla/Istanbul (TR); Sabanci Üniversitesi Nanoteknoloji Arastirma ve Uygulama Merkezi Sunum, 34956 Tuzla/Istanbul (TR); Progin Bilisim Mühendislik ve Danismanlik Hizmetleri Ltd. Sti., 34912 Pendik/Istanbul (TR)
(72) Inventor: KOSAR, Ali, 34956 Tuzla/Istanbul (TR); KUTLU, Özlem, 34956 Tuzla/Istanbul (TR); CETINEL, Sibel, 34956 Tuzla/Istanbul (TR); GHORBANI, Morteza, 34956 Tuzla/Istanbul (TR); TALEBIAN GEVARI, Moein, 34956 Tuzla/Istanbul (TR); ATALAY, Ismail Cem, 34912 Pendik/Istanbul (TR)
(74) Representative: Genç Ilhan, Oznur
(86) International application number: PCT/TR2020/050676
(87) International publication number: WO 2022/025836

(56) References cited:
- WO-A2-2012/151501
- US-A1- 2010 323 342
- MORTEZA GHORBANI ET AL: "Hydrodynamic cavitation in microfluidic devices with roughened surfaces", JOURNAL OF MICROMECHANICS AND MICROENGINEERING, INSTITUTE OF PHYSICS PUBLISHING, BRISTOL, GB, vol. 28, no. 7, 26 April 2018 (2018-04-26), pages 75016, XP020328323, ISSN: 0960-1317, [retrieved on 20180426], DOI: 10.1088/1361-6439/AAB9D0
- ÖZBEY ARZU ET AL: "Inertial focusing of microparticles in curvilinear microchannels with different curvature angles", MICROFLUIDICS AND NANOFLUIDICS, SPRINGER, DE, vol. 22, no. 6, 4 June 2018 (2018-06-04), pages 1 - 16, XP036532433, ISSN: 1613-4982, [retrieved on 20180604], DOI: 10.1007/S10404-018-2082-0

## Description

### Technical Field of the Invention

The invention relates generally to a device and a method in the field of diagnostics and more specifically to a device, an integrated system and a method for detecting circulating tumor cells (CTCs) *in vitro.*

### Background of the Invention

Conventional diagnostic strategies for primary tumor rely on imaging techniques such as Magnetic Resonance Imaging (MRI), Computed Tomography (CT), Magnetic Resonance Spectroscopy (MRS), Positron Emission Tomography (PET), Radiography (X-ray), ultrasonography and tissue biopsy examination under microscope. The efficiency of these techniques highly depends on cancer type / location and usually utilize specific biomarkers identified from genomic or proteomic analysis. However, these techniques can only be used if the tumor is achieved to a certain size and cannot be applied to identify the presence of tumor in early stages. Moreover, the evaluation of molecular biomarkers is usually performed by using primary tumor tissues but this approach does not represent potential diversity and heterogeneity between primary tumor and metastatic lesions [Vignot, B. Besse, F. André, J.-P. Spano, J.-C. Soria, Discrepancies between primary tumor and metastasis: a literature review on clinically established biomarkers, Crit. Rev. Oncol. Hematol. 84 (2012) 301-313].

Circulating Tumor Cells (CTCs) are defined as tumor cells detached from primary tumor site to travel through the vasculatures to vital organs and causes secondary tumors, i.e., metastasis. It is known that a high percentage of cancer patients throughout the world die because of metastatic spread of CTCs and also because of the delay in the diagnosis. Furthermore, CTCs can potentially provide important clinical information that can be used for detecting and monitoring of cancer. Thus, development of detection methods or devices for CTCs will play an important role in early detection of cancer and ultimately enhance effective treatment strategies.

Early diagnosis of cancer is of vital importance not only for general prognosis, but also for the choice of treatment strategies such as chemotherapy, radiotherapy or surgery. Currently, blood and its derivatives such as serum and plasma are the most used biological fluids for diagnosis and mounting evidence indicates that cancer biomarkers in blood can lead to a non-invasive detection approach for cancer as well as the determination of tumor type. However, detection of these biomarkers at certain level in blood is only available at progressive stages of cancer; thereby most of the cancer biomarkers are not sensitive enough for early diagnosis or follow-up of cancer patients. Tissue biopsies are another "gold standard" source for tumor diagnosis, often used to confirm the existence of tumor following of the blood analyses. Nevertheless, biopsy is an invasive procedure for patients and this technique has several limitations.

US 20170128939A1 discloses an integrated system to isolate and diagnose CTCs within a cellular sample includes an isolating mechanism to isolate and trap large biological cells at a detection zone from among the cellular sample based on cells size, and includes a diagnosing mechanism to diagnose CTCs among the trapped large biological cells. The diagnosing mechanism of the disclosed system uses electrical impedance difference between CTCs and blood cells in flow of blood within fabricated micro-channels on silicon. The referred invention isolates CTCs and large blood cells in vertically etched micro-channels and it is integrated with electrodes to detect the trapped cells at the inlet of the channels.

EP 1984030 B1 relates to a method for detecting CTCs in a mammalian subject or a method of diagnosing cancer in a mammalian subject. The disclosed method comprises obtaining a test sample from blood of the mammalian subject; mounting the test sample on a substrate; detecting a first marker in the test sample that selectively binds to the CTCs; detecting a second marker in the test sample that binds to the cell population or a subset of the cell population; and analyzing the cell population detected by the first and second markers to identify and characterize the CTCs.

US 20150185204 A1 discloses a method for diagnosing lung cancer in a subject comprising (a) generating CTC data from a blood sample obtained from the subject based on a direct analysis comprising immunofluorescent staining and morphological characteristics of nucleated cells in the sample, wherein CTCs are identified in context of surrounding nucleated cells based on a combination of the immunofluorescent staining and morphological characteristics; (b) obtaining clinical data for the subject; (c) combining the CTC data with the clinical data to diagnose lung cancer in the subject.

WO 2012151501 provides an apparatus for detecting CTCs by analyzing a biological subject, then diagnosing the cancer development or metastasis status thereof. Said apparatus has micro-devices integrated onto it for carrying out diagnosis atmicroscopic levels, in vivo or in vitro, on the biological subject containing cells. Furthermore, said apparatus comprises a biological fluid delivering system, a preprocessing unit, a recharging unit, a probing and detecting device, and a discharging unit. Additional micro-devices, e.g., a second detection device, can also be includedor integrated into the apparatus for enhanced detection capabilities. However, a microflow restrictive element having an inlet portion of the microfluidic device with an inner cross-section area larger than inner cross-section area of the microflowrestrictive element, and/or generation of cavitation inception based on the disclosed configuration of the microflow restrictive element and the microfluidic device is not suggested in the document.US 20170128939A1 discloses an integrated system to isolate and diagnose CTCs within a cellular sample includes an isolating mechanism to isolate and trap large biological cells at a detection zone from among the cellular sample based on cells size, and includes a diagnosing mechanism to diagnose CTCs among the trapped large biological cells. The diagnosing mechanism of the disclosed system uses electrical impedance difference between CTCs and blood cells in flow of blood within fabricated micro-channels on silicon. The referred invention isolatesCTCs and large blood cells in vertically etched micro-channels and it is integratedwith electrodes to detect the trapped cells at the inlet of the channels.

Current pre-diagnostic technologies for cancer are not available in all first-line health-care premises due to high capital and operational expenses. Additionally, cancer pre-diagnosis via classical laboratory equipment such as centrifuges, special bio-chemical reactors/catalyzers and microscopic scanning/identification requires long processing time. Thus, there is still a need for a device and a method for detecting circulating tumor cells (CTCs) in a biological fluid sample.

The present invention further achieves CTC detection by using a concept based on Cavitation-on-Chip principle. Cavitation is a phenomenon, in which rapid changes of pressure in a liquid lead to the formation of small vapor-filled cavities (i.e., microbubbles) in places, where the pressure is relatively low. The process of beginning of cavitation is called "cavitation inception" that is highly dependent on the thermo-physical properties of the studied liquid.

### Objects of the Invention

Primary object of the present invention is to overcome the abovementioned shortcomings of the prior art.

Another object of the present invention is to provide a device and a method for detecting presence of CTCs in a biological fluid sample without utilizing any special nano/magnetic/optic labeled biomarkers.

Another object of the present invention is to provide a device that can be used repeatedly and also provides reliable results.

Another object of the present invention is to provide a method for diagnosis of cancer in its early stages.

Another object of the present invention is to provide a diagnosis method easy and safe to perform.

Another object of the present invention is to provide a low cost diagnostic device and a method providing real-time monitoring.

Another object of the present invention is to provide a method for detecting CTCs in a biological fluid sample *in vitro.*

### Summary of the Invention

The present invention proposes a device for detecting circulating tumor cells (CTCs) in a biological fluid sample, comprising a microfluidic device having an inlet portion for receiving the biological fluid sample into said microfluidic device, and an outlet portion for discharging the received biological fluid sample from said microfluidic device. Said microfluidic device further houses a micro flow restrictive element extending between the inlet portion and the outlet portion of said microfluidic device. Inner cross-section area of the inlet portion of the microfluidic device is larger than inner cross-section area of said micro flow restrictive element; and this reduction in inner cross-section area between the microfluidic device and the micro flow restrictive element induces a rapid drop in pressure of the biological fluid sample flowing through inlet zone of said micro flow restrictive element to a critical level, which is equal to or below saturated vapor pressure of said fluid sample at room temperature. Additionally, the device comprises an ultrasonic signal processing system including an ultrasonic transmitter for transmitting ultrasound waves on said biological fluid sample while it is flowing through said micro flow restrictive element; an ultrasonic receiver for receiving ultrasound waves reflected from said biological fluid sample; and an ultrasonic signal processor analyzing information obtained from the ultrasonic transmitter and the ultrasonic receiver; and detecting presence of CTCs within the flowing biological fluid sample considering variation of the monitored reflected ultrasound waves due to generation of cavitation inception based on said rapid pressure drop within the micro flow restrictive element.

According to an embodiment, the biological fluid sample is a CTC-enriched blood or serum sample.

According to an embodiment, the biological fluid sample comprises at least one of white blood cells (WBCs), red blood cells (RBCs), CTCs or any combination thereof.

According to an embodiment, hydraulic diameter of micro flow restrictive element is between 10 µm to 250 µm.

According to an embodiment, the device comprises roughness elements formed on inner wall surface of said micro flow restrictive element.

According to an embodiment, height of the roughness elements is selected from 0.01W₂ to 0.1W₂ where the hydraulic diameter of the micro flow restrictive element is 1W₂.

According to an embodiment, the roughness elements are formed on inner wall surface of inlet zone of the micro flow restrictive element.

The present invention further proposes an integrated system for enriching and detecting circulating tumor cells (CTCs) in a biological fluid sample, comprising a microfluidic enrichment device for enriching CTCs in said biological fluid sample comprising a channel having at least a channel inlet and at least a channel outlet for said biological fluid sample flow; and the device according to any one of the embodiments of the present invention.

The present invention further proposes a method for detecting circulating tumor cells (CTCs) in a biological fluid sample, comprising the steps of: a. supplying the biological fluid sample to an inlet portion of microfluidic device housing a micro flow restrictive element where inner cross-section area of the microfluidic device is larger than inner cross-section area of said micro flow restrictive element; b. transmitting ultrasound waves on said biological fluid sample while flowing through said micro flow restrictive element; c. receiving ultrasound waves reflected from said biological fluid sample; d. analyzing information according to values of the transmitted and received ultrasound waves, where variation of the monitored reflected ultrasound waves indicates generation of cavitation inception due to rapid drop in pressure of the biological fluid sample flowing through inlet zone of said micro flow restrictive element, which further indicates presence of CTCs in said biological fluid sample.

According to an embodiment, the detection method further comprises the step of performing a CTC enrichment treatment on said biological fluid sample to obtain a CTC-enriched biological fluid sample, before step (a).

According to an embodiment of the method disclosed according to the present invention, the detection of circulating tumor cells (CTCs) are realized by real-time monitoring the values of the transmitted and received ultrasound waves during flowing of the biological fluid sample through the micro flow restrictive element.

The present invention further proposes use of a device according to any one of the embodiments of the present invention for detecting circulating tumor cells (CTCs) in a biological fluid sample.

The present invention further proposes use of an integrated system according to any one of the embodiments of the present invention for detecting circulating tumor cells (CTCs) in a biological fluid sample.

### Brief Description of the Figures

The figures, whose brief explanation is herewith provided, are solely intended for providing a better understanding of the present invention and are as such not intended to define the scope of protection or the context in which said scope is to be interpreted in the absence of the description.
Fig. 1 shows a simplified plan view of an integrated system including a microfluidic device, an ultrasonic signal processing system and a microfluidic enrichment device for enriching and then detecting circulating tumor cells (CTCs) in a biological fluid sample.
Fig. 2 is a section view of Fig. 1, and shows an embodiment of the microfluidic enrichment device according to the present invention.
Fig. 3 is a section view of Fig. 1, and shows an embodiment of the microfluidic device according to the present invention.
Fig. 4 is a section view of Fig. 1, and shows the microfluidic device having roughness elements formed on inner wall of the micro flow restrictive element according to an embodiment of the present invention.
Fig. 4A is partly enlarged view of Fig. 4, and illustrates a part of the microfluidic device having roughness elements formed on inner wall of the micro flow restrictive element according to an embodiment of the present invention.
Fig. 5 is a cross-section view of micro flow restrictive element showing the roughness elements formed on inner wall of the micro flow restrictive element according to an embodiment of the present invention.
Fig. 6 shows view of the roughness elements according to an embodiment of the present invention, formed on inner wall of the micro flow restrictive element.

### Detailed Description of the Invention

The present invention discloses a device (1), an integrated system (100) including said device (1), and a method for detecting circulating tumor cells (CTCs) in a biological fluid sample *in vitro.*

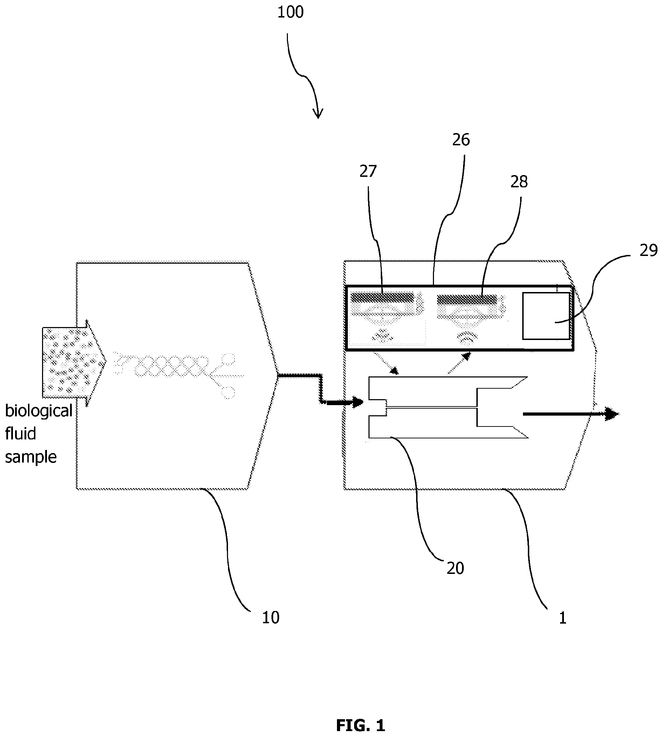

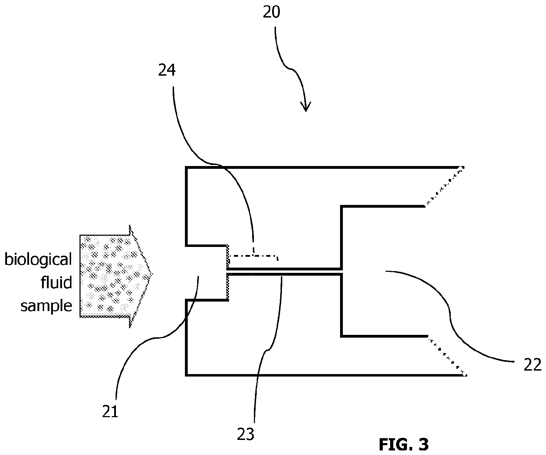

The device (1) according to the present invention is provided for detecting CTCs in a biological fluid sample, and comprises a microfluidic device (20) and an ultrasonic signal processing system (26). Said microfluidic device (20) comprises an inlet portion (21) for receiving the biological fluid sample into said microfluidic device (20), and an outlet portion (22) for discharging the received biological fluid sample from the microfluidic device (20). Said microfluidic device (20) further houses a micro flow restrictive element (23) extending between the inlet portion (21) and the outlet portion (22). Thereby, as shown in Figs. 1, 3 and 4, respectively the inlet portion (21), the micro flow restrictive element (23) and the outlet portion (22) form a fluid passage within said microfluidic device (20) for the biological fluid sample to flow through the micro flow restrictive element (23). The inlet portion (21) of the microfluidic device (20) has an inner cross-section area larger than inner cross-section area of said micro flow restrictive element, which causes a sudden reduction in the inner cross-section area of said fluid passage at the location where the fluid passes from the inlet portion (21) to the micro flow restrictive element (23), i.e., especially to an inlet zone (24) of the micro flow restrictive element (23). This reduction induces pressure of the biological fluid sample flowing through the inlet zone (24) of said micro flow restrictive element (23) to rapidly drop to a critical level, which is equal to or below saturated vapor pressure of said fluid sample at room temperature (about 20 °C).

The rapid drop of pressure of the biological fluid sample to its critical level leads formation of microbubbles (small vapor-filled cavities) in said biological fluid sample while it is flowing through the micro flow restrictive element (23), especially through the inlet zone (24) which is close to the inlet portion (21) where the pressure is relatively low. As will be acknowledged by a skilled person in the art, length of the inlet zone (24) within the micro flow restrictive element (23) can appropriately be calculated, and thus be adjusted especially by considering measured pressure value on the biological fluid sample flowing through said inlet zone (24). Formation of the microbubbles is disclosed by phenomenon called as cavitation inception.

The ultrasonic signal processing system (26) of the device (1) includes an ultrasonic transmitter (27) for transmitting ultrasound waves on said biological fluid sample while it is flowing through said micro flow restrictive element (23); and an ultrasonic receiver (28) for receiving ultrasound waves reflected from said biological fluid sample. Said ultrasonic signal processing system (26) also comprises an ultrasonic signal processor (29) analyzing information obtained from the ultrasonic transmitter (27) and the ultrasonic receiver (28); and detecting presence of CTCs, if any, in the flowing biological fluid sample considering variation of the monitored reflected ultrasound waves due to generation of cavitation inception based on said rapid pressure drop within the micro flow restrictive element (23).

The tested biological fluid sample may be a blood or serum sample. Optionally, the tested biological fluid sample may be CTC-enriched blood or serum sample. Furthermore, the biological fluid sample may comprise at least one of white blood cells (WBCs), red blood cells (RBCs), CTCs or any combination thereof.

Differential response of cavitation inception to micro-level adjustment of environmental conditions (such as liquid pressure) could be used as indicator of altered rheological characteristics. Furthermore, it is known that, rheological characteristics of fluids change when there are solid particles (i.e., cell) floating inside said fluid. In the technique, it is reported that size (diameter, area or volume) of a CTC is considered to be larger than any normal blood cell (WBC, RBC, etc.). As to exemplify, volume of a breast cancer CTC is measured as 851.6 ± 45.8 µm³ , and an Ovarian cancer CTC as 518.3 ± 24.5 µm³ while volume of a RBC is measured as 100.6 ± 4 µm³ , and a WBC as 234.1 ± 4.1 µm³ and Platelet as 10.5 ± 0.5 µm³ by DIC microscope [K. Phillips, A. Kolatkar, K. Rees, R. Rigg, D.Marrinucci,M. Luttgen, K. Bethel, P. Kuhn, O. McCarty, Quantification of cellular volume and sub-cellular density fluctuations: comparison of normal peripheral blood cells and circulating tumor cells identified in a breast cancer patient, Front. Oncol. 2 (2012).]. Area of a breast cancer CTC is measured as 290 ± 200 µm², a colorectal cancer CTC as 186 ± 153 µm², a prostate cancer CTC as 180 ± 145 µm² while area of a WBC is measured as 71 ± 44 µm² by Images from CellSearch^{®} system [S.T. Ligthart, F.A. Coumans, F.-C. Bidard, L.H. Simkens, C.J. Punt, M.R. de Groot, G. Attard, J.S. de Bono, J.-Y. Pierga, L.W. Terstappen, Circulating tumor cells count and morphological features in breast, colorectal and prostate cancer, PLoS One 8 (2013) e67148.]. Thereby, the rheology of the biological fluid sample without CTCs (i.e., healthy sample) is clearly different from that of the biological fluid sample including CTCs because, as exemplified above, the biological fluid sample including CTCs (unhealthy sample) contains cells of a larger size than the healthy sample. Accordingly, compared to the healthy sample, early cavitation inception is observed for the biological fluid sample including CTCs and supercavitation condition appeared sooner where the supercavitation flow regime corresponds to a fully developed cavitating flow. Furthermore, particles in the biological fluid sample are affecting the cavitation inception behavior considerably, especially if they have surface area that contains nano/micro-level irregularities. With this type of irregular surface area, cavitation inception occurs at lower inlet pressures. CTCs are abnormal cells with irregular surfaces, and any biological fluid sample having CTCs and especially CTC-enriched sample is therefore expected to observe early cavitation inception as compared to healthy sample. Consequently, compared to the healthy sample, cavitation inception and hence the generation of microbubbles is observed earlier in the biological fluid sample containing CTCs; and the generated microbubbles are easily detected by the ultrasonic signal processing system (26) according to the present invention.

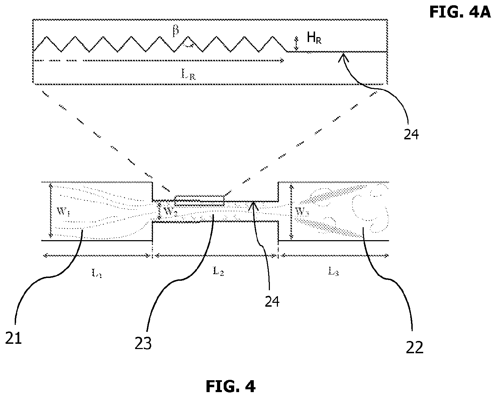

According to a preferred embodiment of the present invention, cross-sectional area of one or more of the inlet portion (21), outlet portion (22), micro flow restrictive element (23) and inlet zone (24) disclosed in the present specification may be rectangular. Accordingly, depth of at least any one of the inlet portion (21), outlet portion (22), micro flow restrictive element (23) and inlet zone (24) may optionally be selected around 50 µm. According to an embodiment, hydraulic diameter of the micro flow restrictive element (W₂) is between 10 µm to 250 µm, and preferably between 150 µm to 250 µm. As will be acknowledged by a skilled person, the hydraulic diameter of the micro flow restrictive element (W₂) may be designed according to the type of the cancer that is required to be detected with said device (1) and accordingly, an optimization may be beneficial in order to realize a precise measurement, taking into account the size of the CTC to be detected, the inlet pressure of the biological fluid sample fed to the inlet portion (21) of the microfluidic device (20) and the hydraulic diameter of the micro flow restrictive element (W₂). According to a preferred embodiment of the present invention, the inlet pressure of the biological fluid sample fed to the inlet portion (21) of the microfluidic device (20) is between 1 to 3 MPa. Optionally, the inlet pressure and outlet pressure of the microfluidic device (20) may be regulated through a programmable pressure controller (not shown). Main velocity of the cavitating flow inside the micro flow restrictive element (23) may optionally be selected/adjusted as to be between 20 to 52 m/s for the inlet pressures of 1 to 3 MPa. Furthermore, the length of the micro flow restrictive element (L₂) extending between the inlet portion (21) and the outlet portion (22) is optionally selected between 0.5 mm and 2 mm which can be designed according to the desired outlet pressure of the microfluidic device (20). In Fig.4, length of the inlet portion (21) is shown with "L₁" and length of the outlet portion (22) is shown with "L₃". According to another embodiment, hydraulic diameter of the inlet portion (W₁) may be selected around 900 µm.

According to an embodiment of the present invention, the micro flow restrictive element (23) is in structure of a micro orifice optionally having a functional inner surface. Said functional inner surface can be exemplified with roughness elements formed on inner wall (25) surface of said micro flow restrictive element (23). Optionally, said roughness elements are formed on inner wall surface of the inlet zone (24). The roughness elements may be selected from micro/nano pin fins, crevices, cavities, mechanically deformed structures, porous materials, sintered wires/meshes, micro/nano structures and any other complex geometries. Particles in the biological fluid sample affect the cavitation inception behavior considerably, especially if said sample is flowing through a fluid passage with an inner surface area containing any roughness elements. The formed roughness elements lead the biological fluid sample having particles such as CTCs to flow by providing earlier inception of cavitating microbubbles as compared to healthy blood sample and by intensifying developed cavitating flows (supercavitation condition). According to an embodiment of the present invention, height of the roughness elements (H_{R}) can be selected from 0.01W₂ to 0.1W₂ where the coefficient "W₂" corresponds to the hydraulic diameter of the micro flow restrictive element (23). Optionally, length of region designed with said roughness elements (L_{R}) can be selected between 0.3L₂ to L₂ where the coefficient "L₂" corresponds to total length of the micro flow restrictive element (23). In Fig. 4, W₃ corresponds to the hydraulic diameter of the outlet portion (22).

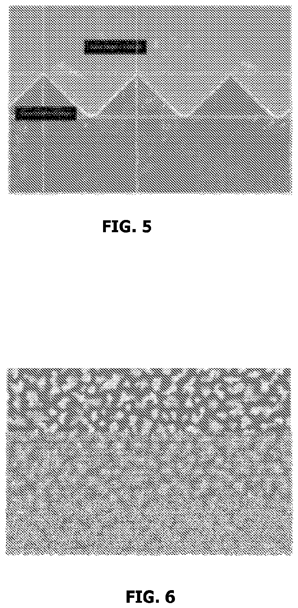

Fig. 5 provides a cross-section view of micro flow restrictive element showing the roughness elements formed on inner wall of the micro flow restrictive element according to an embodiment of the present invention. Furthermore, Fig. 6 provides surface morphology of the roughness elements formed on inner wall of the micro flow restrictive element according to an embodiment of the present invention.

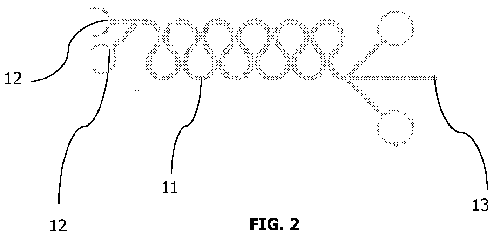

Present invention further discloses an integrated system (100) for enriching and detecting circulating tumor cells (CTCs) in a biological fluid sample. The integrated system (100), provided in Fig.1, comprises the device (1) as provided in any one of the above disclosed embodiments of the present invention, and a microfluidic enrichment device (10) for enriching the CTCs in said biological fluid sample before feeding the same to the device (1). Said microfluidic enrichment device (10) comprises a micro-channel (11) having at least a channel inlet (12) and at least a channel outlet (13) for said biological fluid sample flow. The microfluidic enrichment device (10) is preferably carries out a process for enrichment of the CTCs in biological fluid sample (if any) as to provide typically 1×10³ to 3×10⁵ CTC cells/mL of the sample. As an embodiment, said microfluidic enrichment device (10) may be any efficient device provided for CTC enrichment. The microchannel may be selected from various types of channel geometries including straight, spiral, serpentine, curvilinear and straight channel with contraction-expansion arrays in inertial microfluidics. Preferably, the microchannel is curvilinear as shown in Figs. 1 and 2. On the other hand, the microfluidic enrichment device (10) may be selected from the categories of active or passive separation depending on its energy usage. Active techniques require external forces such as magnetic, dielectric, and acoustic to separate particles/cells, while passive techniques utilize mainly hydrodynamic forces.

According to an embodiment of the present invention, said integrated system (100) may be designed as a Lab-on-Chip (LOC) Device.

The present invention further discloses a method for detecting circulating tumor cells (CTCs) in a biological fluid sample, comprising the steps of:
a. supplying the biological fluid sample to the inlet portion (21) of the microfluidic device (20) housing the micro flow restrictive element (23) where inner cross-section area of the microfluidic device is larger than inner cross-section area of said micro flow restrictive element;
b. transmitting ultrasound waves on said biological fluid sample while it is flowing through said micro flow restrictive element (23);
c. receiving ultrasound waves reflected from said biological fluid sample;
d. analyzing information according to values of the transmitted and received ultrasound waves, where variation of the monitored reflected ultrasound waves indicates generation of cavitation inception due to rapid drop in pressure of the biological fluid sample flowing through inlet zone (24) of said micro flow restrictive element (23), which further indicates presence of CTCs in said biological fluid sample.

According to an embodiment of the present invention the CTC detection method further comprises a step of performing a CTC enrichment treatment on said biological fluid sample to obtain a CTC-enriched biological fluid sample, before supplying the same to the inlet portion (21) of the microfluidic device (20).

The analyzing step of the proposed CTC detection method is performed by the ultrasonic signal processing system (26) of the device (1) according to the present invention. Thereby, the CTC detection method may optionally comprise a step regarding to detection of CTCs by real-time monitoring the values of the transmitted and received ultrasound waves during flowing of the biological fluid sample through the micro flow restrictive element (23). As disclosed above, analysing step is realized by considering variation, if any, of the monitored reflected ultrasound waves due to generation of cavitation inception or supercavitation condition based on said rapid pressure drop within the micro flow restrictive element (23).

The present invention further discloses the use of the device (1) and also the integrated system (100) according to the present invention for in vitro detection of CTCs in a biological fluid sample.

Reference to "embodiment" throughout this specification means that a particular feature, structure or characteristic described in connection with the embodiment is included in at least one embodiment of the present invention. Thus, throughout this specification, the phrases "in one embodiment" or "in an embodiment" may not necessarily all referring to the same embodiment, but also may. For example, in the following claims, any of the claimed embodiments can be used in any combination.

### List of reference numerals:

- 1: device
- 10: microfluidic enrichment device
- 11: channel
- 12: channel inlet
- 13: channel outlet
- 20: microfluidic device
- 21: inlet portion
- 22: outlet portion
- 23: micro flow restrictive element
- 24: inlet zone
- 25: inner wall
- 26: ultrasonic signal processing system
- 27: ultrasonic transmitter
- 28: ultrasonic receiver
- 29: ultrasonic signal processor
- 100: integrated system

- H_{R}: height of the roughness elements
- L_{R}: length of region designed with said roughness elements
- L₁: length of the inlet portion
- L₂: length of the micro flow restrictive element
- L₃: length of the outlet portion
- W₁: hydraulic diameter of the inlet portion
- W₂: hydraulic diameter of the micro flow restrictive element
- W₂: hydraulic diameter of the outlet portion

## Claims

1. A device (1) for detecting circulating tumor cells (CTCs) in a biological fluid sample, comprising
- a microfluidic device (20) having an inlet portion (21) for receiving the biological fluid sample into said microfluidic device (20), and an outlet portion (22) for discharging the received biological fluid sample from said microfluidic device (20); **characterized in that** said microfluidic device (20) further houses a micro flow restrictive element (23) extending between the inlet portion (21) and the outlet portion (22) of said microfluidic device (20); wherein inner cross-section area of the inlet portion of the microfluidic device (20) is larger than inner cross-section area of said micro flow restrictive element; and this reduction in inner cross-section area between the microfluidic device (20) and the micro flow restrictive element (23) configured to induce a rapid drop in pressure of the biological fluid sample flowing through inlet zone (24) of said micro flow restrictive element (23) to a critical level, which is equal to or below saturated vapor pressure of said fluid sample at room temperature;
- an ultrasonic signal processing system (26) including:
an ultrasonic transmitter (27) configured for transmitting ultrasound waves on said biological fluid sample while it is flowing through said micro flow restrictive element (23);
an ultrasonic receiver (28) configured for receiving ultrasound waves reflected from said biological fluid sample; and
an ultrasonic signal processor (29) configured for analyzing information obtained from the ultrasonic transmitter (27) and the ultrasonic receiver (28) and configured for detecting presence of CTCs within the flowing biological fluid sample by considering variation of the monitored reflected ultrasound waves due to generation of cavitation inception based on said rapid pressure drop within the micro flow restrictive element (23).

2. The device (1) according to claim 1, wherein the biological fluid sample is a CTC-enriched blood or serum sample.

3. The device (1) according to claim 1 or 2, wherein the biological fluid sample comprises at least one of white blood cells (WBCs), red blood cells (RBCs), CTCs or any combination thereof.

4. The device (1) according to any one of the claims 1 to 3, wherein hydraulic diameter of the micro flow restrictive element (23) is between 10 µm to 250 µm.

5. The device (1) according to any one of the claims 1 to 4, comprising roughness elements formed on inner wall (25) surface of said micro flow restrictive element (23).

6. The device (1) according to claim 5, wherein height of the roughness elements (H_{R}) is selected from 0.01W₂ to 0.1 W₂ where the hydraulic diameter of the micro flow restrictive element (23) is 1 W₂.

7. The device (1) according to claim 5 or 6, wherein the roughness elements are formed on inner wall surface of inlet zone (24) of the micro flow restrictive element (23).

8. An integrated system (100) for enriching and detecting circulating tumor cells (CTCs) in a biological fluid sample, comprising
a microfluidic enrichment device (10) configured for enriching the circulating tumor cells (CTCs) in said biological fluid sample comprising a channel (11) having at least a channel inlet (12) and at least a channel outlet (13) for said biological fluid sample flow; and
the device (1) according to any one of the claims 1 to 7.

9. A method for detecting circulating tumor cells (CTCs) in a biological fluid sample, **characterized by** comprising the steps of:
a. supplying the biological fluid sample to an inlet portion (21) of microfluidic device (20) housing a micro flow restrictive element (23) extending between said inlet portion (21) and an outlet portion (22) of said microfluidic device (20) where inner cross-section area of the microfluidic device is larger than inner cross-section area of said micro flow restrictive element, and this reduction in inner cross-section area between the microfluidic device (20) and the micro flow restrictive element (23) induces a rapid drop in pressure of the biological fluid sample flowing through the inlet zone (24) of said micro flow restrictive element (23) to a critical level, which is equal to or below saturated vapor pressure of said fluid sample at room temperature;
b. transmitting ultrasound waves on said biological fluid sample while flowing through said micro flow restrictive element (23);
c. receiving ultrasound waves reflected from said biological fluid sample;
d. analyzing information according to values of the transmitted and received ultrasound waves, where variation of the monitored reflected ultrasound waves indicates generation of cavitation inception due to rapid drop in pressure of the biological fluid sample flowing through inlet zone (24) of said micro flow restrictive element (23), which further indicates presence of CTCs in said biological fluid sample.

10. A method according to claim 9 further comprising the step of performing a CTC enrichment treatment on said biological fluid sample to obtain a CTC-enriched biological fluid sample, before step (a).

11. A method according to claim 9 or 10 wherein the detection of circulating tumor cells (CTCs) are realized by real-time monitoring the values of the transmitted and received ultrasound waves during flowing of the biological fluid sample through the micro flow restrictive element (23).

12. Use of a device (1) according to any one of the claim 1-7 for detecting circulating tumor cells (CTCs) in a biological fluid sample.

13. Use of an integrated system (100) according to claim 8 for detecting circulating tumor cells (CTCs) in a biological fluid sample.

## Patentansprüche

1. Vorrichtung (1) zur Erkennung zirkulierender Tumorzellen (CTCs) in einer biologischen Flüssigkeitsprobe, umfassend
- eine mikrofluidische Vorrichtung (20) mit einem Einlassabschnitt (21) zum Aufnehmen der biologischen Flüssigkeitsprobe in die mikrofluidische Vorrichtung (20) und einem Auslassabschnitt (22) zum Abgeben der aufgenommenen biologischen Flüssigkeitsprobe aus der mikrofluidischen Vorrichtung (20); **dadurch gekennzeichnet, dass** die mikrofluidische Vorrichtung (20) ferner ein Mikroelement zur Strömungsdrosselung (23) aufweist, das sich zwischen dem Einlassabschnitt (21) und dem Auslassabschnitt (22) der mikrofluidischen Vorrichtung (20) erstreckt; wobei die innere Querschnittsfläche des Einlassabschnitts der mikrofluidischen Vorrichtung (20) größer ist als die innere Querschnittsfläche des Mikroelements zur Strömungsdrosselung; und diese Verringerung der inneren Querschnittsfläche zwischen der mikrofluidischen Vorrichtung (20) und dem Mikroelement zur Strömungsdrosselung (23) so konfiguriert ist, dass sie einen schnellen Druckabfall der durch die Einlasszone (24) des Mikroelements zur Strömungsdrosselung (23) fließenden biologischen Flüssigkeitsprobe auf ein kritisches Niveau induziert, das gleich dem Sättigungsdampfdruck der Flüssigkeitsprobe bei Raumtemperatur oder darunter ist;
- ein System zur Verarbeitung von Ultraschallsignalen (26), das Folgendes umfasst:
einen Ultraschallsender (27), der so konfiguriert ist, dass er Ultraschallwellen auf die biologische Flüssigkeitsprobe sendet, während diese durch das Mikroelement zur Strömungsdrosselung (23) fließt;
einen Ultraschallempfänger (28), der für den Empfang von Ultraschallwellen konfiguriert ist, die von der biologischen Flüssigkeitsprobe reflektiert werden; und
einen Ultraschallsignalprozessor (29), der so konfiguriert ist, dass er die vom Ultraschallsender (27) und vom Ultraschallempfänger (28) erhaltenen Informationen analysiert, und der so konfiguriert ist, dass er das Vorhandensein von CTCs in der fließenden biologischen Flüssigkeitsprobe nachweist, indem er die Veränderung der überwachten reflektierten Ultraschallwellen aufgrund der Erzeugung von Kavitationseintritt auf der Grundlage des schnellen Druckabfalls im Mikroelement zur Strömungsdrosselung (23) berücksichtigt.

2. Vorrichtung (1) nach Anspruch 1, wobei die Probe der biologischen Flüssigkeit eine mit CTC angereicherte Blut- oder Serumprobe ist.

3. Die Vorrichtung (1) nach Anspruch 1 oder 2, wobei die Probe der biologischen Flüssigkeit mindestens eines der folgenden Elemente umfasst: weiße Blutzellen (WBCs), rote Blutzellen (RBCs), CTCs oder eine beliebige Kombination davon.

4. Vorrichtung (1) nach einem der Ansprüche 1 bis 3, wobei der hydraulische Durchmesser des Mikroelements zur Strömungsdrosselung (23) zwischen 10 µm und 250 µm liegt.

5. Vorrichtung (1) nach einem der Ansprüche 1 bis 4, mit Rauhigkeitselementen, die auf der Oberfläche der Innenwand (25) des Mikroelements zur Strömungsdrosselung (23) ausgebildet sind.

6. Vorrichtung (1) nach Anspruch 5, wobei die Höhe der Rauhigkeitselemente (H_{R}) zwischen 0,01 W₂ und 0,1 W₂ gewählt wird, wobei der hydraulische Durchmesser des Mikroelements zur Strömungsdrosselung (23) 1 W₂ beträgt.

7. Vorrichtung (1) nach Anspruch 5 oder 6, wobei die Rauhigkeitselemente auf der Innenwandfläche der Einlasszone (24) des Mikroelements zur Strömungsdrosselung (23) ausgebildet sind.

8. Ein integriertes System (100) zur Anreicherung und zur Erkennung zirkulierender Tumorzellen (CTCs) in einer biologischen Flüssigkeitsprobe, umfassend
eine mikrofluidische Anreicherungsvorrichtung (10), die zum Anreichern der zirkulierenden Tumorzellen (CTCs) in der biologischen Flüssigkeitsprobe konfiguriert ist, umfassend einen Kanal (11) mit mindestens einem Kanaleinlass (12) und mindestens einem Kanalauslass (13) für den Fluss der biologischen Flüssigkeitsprobe; und
die Vorrichtung (1) nach einem der Ansprüche 1 bis 7.

9. Verfahren zur Erkennung zirkulierender Tumorzellen (CTCs) in einer biologischen Flüssigkeitsprobe, **dadurch gekennzeichnet, dass** es die folgenden Schritte umfasst:
a. Zuführen der biologischen Flüssigkeitsprobe zu einem Einlassabschnitt (21) einer mikrofluidischen Vorrichtung (20), die ein Mikroelement zur Strömungsdrosselung (23) aufnimmt, das sich zwischen dem Einlassabschnitt (21) und einem Auslassabschnitt (22) der mikrofluidischen Vorrichtung (20) erstreckt, wobei die innere Querschnittsfläche der mikrofluidischen Vorrichtung größer ist als die innere Querschnittsfläche des Mikroelements zur Strömungsdrosselung, und diese Verringerung der inneren Querschnittsfläche zwischen der mikrofluidischen Vorrichtung (20) und dem Mikroelement zur Strömungsdrosselung (23) einen schnellen Druckabfall der durch die Einlasszone (24) des Mikroelements zur Strömungsdrosselung (23) fließenden biologischen Flüssigkeitsprobe auf ein kritisches Niveau induziert, das gleich dem Sättigungsdampfdruck der Flüssigkeitsprobe bei Raumtemperatur oder darunter ist;
b. Sendung von Ultraschallwellen auf die biologische Flüssigkeitsprobe, während sie durch das Mikroelement zur Strömungsdrosselung (23) fließt;
c. Empfang von Ultraschallwellen, die von der biologischen Flüssigkeitsprobe reflektiert werden;
d. Analysieren von Informationen entsprechend den Werten der gesendeten und empfangenen Ultraschallwellen, wobei die Veränderung der überwachten reflektierten Ultraschallwellen die Erzeugung von Kavitationseintritt aufgrund eines schnellen Druckabfalls der biologischen Flüssigkeitsprobe, die durch die Einlasszone (24) des Mikroelements zur Strömungsdrosselung (23) fließt, anzeigt, was ferner das Vorhandensein von CTCs in der biologischen Flüssigkeitsprobe anzeigt.

10. Verfahren nach Anspruch 9, umfassend ferner den Schritt der Durchführung einer CTC-Anreicherungsbehandlung an der biologischen Flüssigkeitsprobe vor Schritt (a), um eine CTCangereicherte biologische Flüssigkeitsprobe zu erhalten.

11. Verfahren nach Anspruch 9 oder 10, wobei die Erkennung zirkulierender Tumorzellen (CTCs) durch Echtzeitüberwachung der Werte der gesendeten und empfangenen Ultraschallwellen während des Durchflusses der biologischen Flüssigkeitsprobe durch das Mikroelement zur Strömungsdrosselung (23) erfolgt.

12. Verwendung einer Vorrichtung (1) nach einem der Ansprüche 1-7 zur Erkennung zirkulierender Tumorzellen (CTCs) in einer biologischen Flüssigkeitsprobe.

13. Verwendung eines integrierten Systems (100) nach Anspruch 8 zur Erkennung zirkulierender Tumorzellen (CTCs) in einer biologischen Flüssigkeitsprobe.

## Revendications

1. - Dispositif (1) de détection de cellules tumorales circulantes (CTC) dans un échantillon de fluide biologique, comprenant
- un dispositif microfluidique (20) ayant une partie d'entrée (21) destinée à recevoir l'échantillon de fluide biologique dans ledit dispositif microfluidique (20), et une partie de sortie (22) destinée à évacuer l'échantillon de fluide biologique reçu dudit dispositif microfluidique (20) ; **caractérisé par le fait que** ledit dispositif microfluidique (20) contient en outre un élément de restriction de micro-écoulement (23) s'étendant entre la partie d'entrée (21) et la partie de sortie (22) dudit dispositif microfluidique (20) ; dans lequel la surface de section transversale interne de la partie d'entrée du dispositif microfluidique (20) est plus grande que la surface de section transversale interne dudit élément de restriction de micro-écoulement ; et cette réduction de la surface de section transversale interne entre le dispositif microfluidique (20) et l'élément de restriction de micro-écoulement (23) configurée pour induire une chute rapide de la pression de l'échantillon de fluide biologique s'écoulant à travers la zone d'entrée (24) dudit élément de restriction de micro-écoulement (23) jusqu'à un niveau critique, qui est égal ou inférieur à la pression de vapeur saturée dudit échantillon de fluide à température ambiante ;
- un système de traitement de signal ultrasonore (26) comprenant :
un émetteur ultrasonore (27) configuré pour transmettre des ondes ultrasonores sur ledit échantillon de fluide biologique pendant qu'il s'écoule à travers ledit élément de restriction de micro-écoulement (23) ;
un récepteur ultrasonore (28) configuré pour recevoir des ondes ultrasonores réfléchies à partir dudit échantillon de fluide biologique ; et
un processeur de signal ultrasonore (29) configuré pour analyser les informations obtenues à partir de l'émetteur ultrasonore (27) et le récepteur ultrasonore (28) et configuré pour détecter la présence de CTC à l'intérieur de l'échantillon de fluide biologique en écoulement en tenant compte de la variation des ondes ultrasonores réfléchies surveillées en raison de la génération d'un début de cavitation sur la base de ladite chute de pression rapide à l'intérieur de l'élément de restriction de micro-écoulement (23) .

2. - Dispositif (1) selon la revendication 1, dans lequel l'échantillon de fluide biologique est un échantillon de sang ou de sérum enrichi en CTC.

3. - Dispositif (1) selon l'une des revendications 1 ou 2, dans lequel l'échantillon de fluide biologique comprend au moins l'un parmi des globules blancs (WBC), des globules rouges (RBC), des CTC ou toute combinaison de ceux-ci.

4. - Dispositif (1) selon l'une quelconque des revendications 1 à 3, dans lequel le diamètre hydraulique de l'élément de restriction de micro-écoulement (23) est entre 10 µm et 250 µm.

5. - Dispositif (1) selon l'une quelconque des revendications 1 à 4, comprenant des éléments de rugosité formés sur la surface de la paroi interne (25) dudit élément de restriction de micro-écoulement (23).

6. - Dispositif (1) selon la revendication 5, dans lequel la hauteur des éléments de rugosité (H_{R}) est choisie entre 0,01 W₂ et 0,1 W₂, où le diamètre hydraulique de l'élément de restriction de micro-écoulement (23) est de 1 W2.

7. - Dispositif (1) selon l'une des revendications 5 ou 6, dans lequel les éléments de rugosité sont formés sur la surface de paroi interne de la zone d'entrée (24) de l'élément de restriction de micro-écoulement (23).

8. - Système intégré (100) pour enrichir et détecter des cellules tumorales circulantes (CTC) dans un échantillon de fluide biologique, comprenant un dispositif d'enrichissement microfluidique (10) configuré pour enrichir les cellules tumorales circulantes (CTC) dans ledit échantillon de fluide biologique comprenant un canal (11) ayant au moins une entrée de canal (12) et au moins une sortie de canal (13) pour ledit écoulement d'échantillon de fluide biologique ; et
le dispositif (1) selon l'une quelconque des revendications 1 à 7.

9. - Procédé de détection de cellules tumorales circulantes (CTC) dans un échantillon de fluide biologique, **caractérisé par le fait qu'**il comprend les étapes suivantes
a. fournir l'échantillon de fluide biologique à une partie d'entrée (21) d'un dispositif microfluidique (20) contenant un élément de restriction de micro-écoulement (23) s'étendant entre ladite partie d'entrée (21) et une partie de sortie (22) dudit dispositif microfluidique (20) où la surface de section transversale interne du dispositif microfluidique est plus grande que la surface de section transversale interne dudit élément de restriction de micro-écoulement, et cette réduction de la surface de section transversale interne entre le dispositif microfluidique (20) et l'élément de restriction de micro-écoulement (23) induit une chute rapide de la pression de l'échantillon de fluide biologique s'écoulant à travers la zone d'entrée (24) dudit élément de restriction de micro-écoulement (23) jusqu'à un niveau critique, qui est égal ou inférieur à la pression de vapeur saturée dudit échantillon de fluide à température ambiante ;
b. transmettre des ondes ultrasonores sur ledit échantillon de fluide biologique pendant qu'il s'écoule à travers ledit élément de restriction de micro-écoulement (23) ;
c. recevoir des ondes ultrasonores réfléchies à partir dudit échantillon de fluide biologique ;
d. analyser les informations en fonction des valeurs des ondes ultrasonores transmises et reçues, où la variation des ondes ultrasonores réfléchies surveillées indique la génération d'un début de cavitation en raison d'une chute de pression rapide de l'échantillon de fluide biologique s'écoulant à travers la zone d'entrée (24) dudit élément de restriction de micro-écoulement (23), ce qui indique en outre la présence de CTC dans ledit échantillon de fluide biologique.

10. - Procédé selon la revendication 9 comprenant en outre l'étape d'exécution d'un traitement d'enrichissement en CTC sur ledit échantillon de fluide biologique pour obtenir un échantillon de fluide biologique enrichi en CTC, avant l'étape (a).

11. - Procédé selon l'une des revendications 9 ou 10, dans lequel la détection de cellules tumorales circulantes (CTC) est réalisée par surveillance en temps réel des valeurs des ondes ultrasonores transmises et reçues pendant l'écoulement de l'échantillon de fluide biologique à travers l'élément de restriction de micro-écoulement (23).

12. - Utilisation d'un dispositif (1) selon l'une quelconque des revendications 1 à 7 pour détecter des cellules tumorales circulantes (CTC) dans un échantillon de fluide biologique.

13. - Utilisation d'un système intégré (100) selon la revendication 8 pour détecter des cellules tumorales circulantes (CTC) dans un échantillon de fluide biologique.
